# EUROPÄISCHE PATENTSCHRIFT

(11) **EP 1 173 249 B1**
(45) Veröffentlichungstag und Bekanntmachung des Hinweises auf die Patenterteilung: **20.08.2003**
(21) Anmeldenummer: 00926957.2
(22) Anmeldetag: 15.04.2000
(51) Int. Cl.: A61M 25/06, A61M 31/00, A61B 1/00, A61B 17/34

(54) **INTERVENTIONSVORRICHTUNG**
INTERVENTION DEVICE
DISPOSITIF D'INTERVENTION

(30) Priorität: 23.04.1999 DE 19918483
(43) Veröffentlichungstag der Anmeldung: 23.01.2002
(73) Patentinhaber: Gratzki, Torsten, 45239 Essen (DE)
(72) Erfinder: GRATZKI, Torsten, D-45239 Essen (DE); SEIBEL, Rainer, M., M., D-45134 Essen (DE); DEERBERG, Jens, D-44789 Bochum (DE)
(74) Vertreter: Schüll, Gottfried, Dipl.-Phys.
(86) Internationale Anmeldenummer: EP0003436
(87) Internationale Veröffentlichungsnummer: WO00064526

(56) Entgegenhaltungen:
- EP-A- 0 253 990
- WO-A-99/16496
- US-A- 4 013 080
- US-A- 5 304 144
- US-A- 5 429 598
- US-A- 5 498 241
- US-A- 5 885 251

## Beschreibung

Die Erfindung betrifft eine Interventionsvorrichtung mit einer Nadel und einer ersten Handhabe, wobei die erste Handhabe mit der Nadel fest verbunden ist, sowie mit einer zweiten Handhabe.

Aus dem Stand der Technik sind eine Vielzahl von Interventionseinrichtungen zum Einsatz am menschlichen oder tierischen Körper bekannt, die in unterschiedlicher Art und zu unterschiedlichen Zwecken angewandt werden. Gemeinsam ist diesen Interventionsvorrichtungen eine Nadel, die zur Intervention in den insbesondere menschlichen Körper eingebracht wird. Zur Einbringung dieser Nadel weist die Interventionsvorrichtung eine erste Handhabe auf, mit der die behandelnde Person das Einbringen der Nadel in das Gewebe steuert. Hierzu ist die erste Handhabe mit der Nadel in dem Sinne fest verbunden, daß die Verbindung zwischen der Nadel und der Handhabe hinreichend fest ist, um die gewünschte Steuerung der Nadel beim Eindringen in das Gewebe zu ermöglichen. Hierzu kann die Handhabe mit der Nadel entweder dauerhaft, d.h. nicht lösbar, fest verbunden sein aber auch beispielsweise reibschlüssig, d.h. nach dem Einführen lösbar, verbunden sein.

Die bekannten Interventionsvorrichtungen werden, wie bereits erwähnt, zu verschiedenen Zwecken eingesetzt. Beispielsweise werden diese Interventionsvorrichtungen im Rahmen einer Schmerz- und Tumortherapie als Infusionsnadeln eingesetzt, die zunächst bis in den zu therapierenden Bereich vorgeschoben werden, woraufhin anschließend punktgenau eine medizinisch wirksame Substanz injiziert wird. Außerdem werden die bekannten Interventionsvorrichtungen in bestimmten Ausgestaltungen zur Gewinnung von Gewebsproben aus dem menschlichen oder tierischen Körper zur sogenannten Biopsie eingesetzt. Hierbei wird die Nadel wiederum möglichst punktgenau in das zu untersuchende bzw. zu therapierende Gewebe eingebracht, woraufhin in der Nadel ein Mechanismus ausgelöst wird, der aus dem umgebenden Gewebe einen Gewebe kern entnimmt.

Bei den bekannten Interventionsvorrichtungen weist, wie bereits erwähnt, die Nadel, in der Regel an ihrem der Spitze fernliegenden Ende, eine Handhabe auf, die den Angriffspunkt für die behandelnde Person beim Eindringen in das Gewebe darstellt. Üblicherweise wird die Nadel dabei von der behandelnden Person in der Nähe des Einstichs in das Gewebe mit zwei Fingern der nicht an der Handhabe angreifenden Hand der behandelnden Person geführt, um so ein möglichst gezieltes Vordringen der Nadel in das Gewebe zu ermöglichen. Diese Führung der Nadel mit zwei Fingern einer Hand in der Nähe des Einstichpunktes ist unter verschiedenen Gesichtspunkten problematisch. Zum einen ergeben sich aufgrund des naturbedingt elastischen Formschlusses zwischen den menschlichen Fingern und der in der Regel sehr dünnen. Nadel nur eingeschränkte Möglichkeiten bei der Steuerung der Eindringrichtung. Zum anderen ergeben sich auch septische Probleme, da nur schwer zu gewährleisten ist, daß die angreifende Hand, bzw, der diese Hand umgebende Handschuh völlig keimfrei ist.

Darüber hinaus sind aus der US Patentschrift US 5,304,144 Katheterkanülen bekannt, welche als Einführhilfe eine zweite Handhabe in Form von biegsamen Kunststoffflügeln aufweist. Allerdings wird beim Greifen der Handhabe zur Unterstützung des Einführens der Katheternadel die Katheternadel fest gegriffen, so dass die Handhabe nicht auf der Nadel verschiebbar ist.

Ausgehend von dem geschilderten Stand der Technik liegt der vorliegenden Erfindung die Aufgabe zugrunde, eine Interventionsvorrichtung zur Verfügung zu stellen, die eine optimale Steuerbarkeit beim Eindringen in das Gewebe und gleichzeitig optimale septische Verhältnisse gewährleistet.

Erfindungsgemäß ist die zuvor hergeleitete und aufgezeigte Aufgabe dadurch gelöst daß die zweite Handhabe beim Einführen in das Gewebe in Längsrichtung verschiebbar auf der Nadel angeordnet ist. Mit der erfindungsgemäßen Maßnahme ist gewährleistet, daß sich die zweite Handabe bei der Handhabung der erfindungsgemäßen Interventionsvorrichtung in der Nähe des Einstichspunktes der Nadel befindet, die Nadel durch diese zweite Handhabe hindurch geführt wird und dabei aufgrund der deutlich vergrößerten Angriffsfläche der führenden Hand an der zweiten Handhabe und damit an der Nadel eine wesentlich verbesserte Steuerung des Eindringens der Nadel in das Gewebe gewährleistet und gleichzeitig, da kein direkter Kontakt zwischen der Handhabe angreifenden Hand und der Nadel entsteht, optimale aseptische Verhältnisse vorliegen.

Eine weitere ganz wesentliche Problematik der bekannten Konstruktionen von Interventionsvorrichtungen besteht darin, daß diese häufig in Verbindung mit einer bildgebenden Diagnostik eingesetzt werden, die in Echtzeit das Vordringen der Nadel im Gewebe abbildet, so daß eine punktgenaue Steuerung des Vordringens der Nadel zum erkrankten Gewebe möglich ist. Als Verfahren zur bildgebenden Diagnostik wird hierbei regelmäßig die Computertomographie, die in Echtzeit Bilder über das Vordingen der Nadel in das Gewebe liefert, eingesetzt. Problematisch ist hierbei, daß die elektromagnetische Röntgenstrahlung, die bei der Computertomographie zur Bildgewinnung eingesetzt wird, zwar auf einen eng begrenzten Bereich fokussierbar ist, dieser Bereich sich prinzipbedingt jedoch zumindest teilweise mit dem Einstichpunkt der Nadel in das Gewebe überlappt. Da bei den bekannten Interventionsvorrichtungen die Nadel in der Nähe des Einstichpunkts über zwei Finger einer Hand der behandelnden Person gesteuert wird, sind zumindest die Fingerkuppen der behandelnden Person einer erhöhten Strahlenbelastung durch die elektromagnetische Röntgenstrahlung ausgesetzt. Zwar ist die Intensität dieser Röntgenstrahlung bei der Computertomographie relativ gering es ergibt sich jedoch aufgrund der Häufigkeit von mittels Computertomographien gesteuerten Interventionen auf Dauer eine nicht zu vertretende Strahlenbelastung der Finger der behandelnden Personen. Zur Lösung dieser Problematik ist die erfindungsgemäße Interventionsvorrichtung gemäß einer ersten besonders vorteilhaften Ausgestaltung dadurch weitergebildet, daß die erste und/oder zweite Handhabe radial zur Achse der Nadel eine Verlängerung aufweisen. Über eine derartige Verlängerung der Handhabe in radialer Richtung um etwa 2 bis 10cm, vorzugsweise etwa 5cm, wird gewährleistet, daß die behandelnde Person einerseits eine optimale Kontrolle über das Eindringen der Nadel in das Gewebe hat andererseits die Finger der Hände der behandelnden Person an den Handhaben in einem Bereich angreifen, der nur noch einer sehr geringen, vertretbaren Strahlenbelastung verursacht durch die bildgebende Diagnostik ausgesetzt ist.

Bei der Verwendung der erfindungsgemäßen Interventionsvorrichtung im Rahmen einer über eine bildgebende Diagnostik gesteuerte Intervention ist es weiter vorteilhaft, daß das Material der ersten und/oder zweiten Handhabe transparent für elektromagnetische Strahlung ist, so daß gewährleistet ist, daß die Handhabe im Bild bei der Steuerung des Eindringens unsichtbar ist, d.h. die Betrachtung nicht stört.

Die auf der Nadel verschiebbare zweite Handhabe wird in dem Augenblick überflüssig, in dem die Interventionsvorrichtung in den gewünschten Gewebebereich eingebracht wurde und nicht weiter bewegt werden soll. In diesem Zustand belastet die zweite Handhabe unter Umständen die Einstichstelle durch ihr Gewicht und ist für den Patienten eben aus diesem Grund unangenehm. Um dies zu vermeiden, weist die zweite Handhabe der erfindungsgemäßen Interventionsvorrichtung vorzugsweise einen Längsschlitz zum Einbringen der Nadel in die axiale Führung der zweiten Handhabe aus einer Richtung radial zur Achse der Nadel auf. Hierdurch wird gewährleistet, daß die zweite Handhabe beispielsweise nach dem Vordringen der Interventionsvorrichtung an den gewünschten Ort abnehmbar ist. Dies hat außerdem den Vorteil, daß die zweite Handhabe mehrfach wiederverwendet werden kann.

Bei der Verwendung der erfindungsgemäßen Interventionsvorrichtung im Rahmen der Schmerz- und Tumortherapie ist gemäß einer weiteren Ausgestaltung der Erfindung in der Nadel eine entfernbare Innenkanüle angeordnet, die beim Eindringen der Nadel in das Gewebe verhindert, daß in die Nadel Gewebsflüssigkeit- und -material eindringt.

Zur Herausnahme der Innenkanüle aus der Nadel nach dem Vordringen der Nadel in das zu behandelnde Gewebe ist die Innenkanüle vorteilhafterweise an einer dritten Handhabe befestigt, wobei die dritte Handhabe mit der ersten Handhabe lösbar verbunden ist.

Es gibt nun eine Vielzahl von Möglichkeiten die erfindungsgemäße Interventionsvorrichtung auszugestalten und weiterzubilden. Hierzu wird beispielsweise verwiesen einerseits auf die dem Patentanspruch 1 nachgeordneten Patentansprüche andererseits auf die Beschreibung eines Ausführungsbeispiels in Verbindung mit der Zeichnung. In der Zeichnung zeigt
- Fig. 1: ein Ausführungsbeispiel einer erfindungsgemäßen Interventionsvorrichtung in einer Seitenansicht und
- Fig. 2: das Ausführungsbeispiel einer erfindungsgemäßen Interventionsvorrichtung in einer perspektivischen Ansicht.

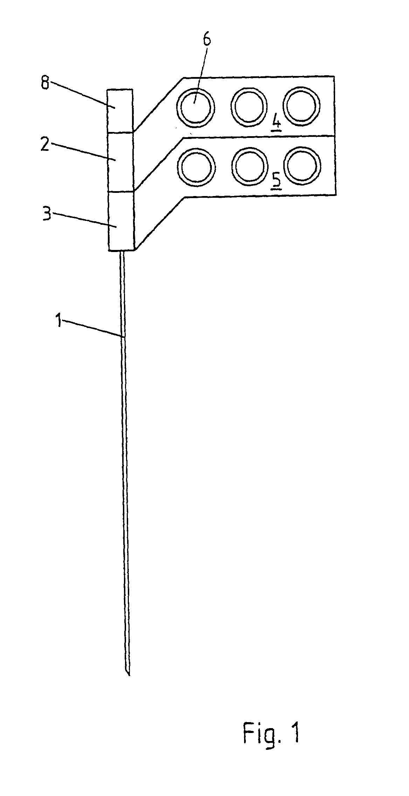

Das in Fig. 1 dargestellte Ausführungsbeispiel einer erfindungsgemäßen Interventionsvorrichtung weist eine Nadel 1 und eine erste Handhabe 2 auf, wobei die erste Handhabe 2 mit der Nadel 1 zumindest so fest verbunden ist, daß die Übertragung einer zum Einbringen in das Gewebe notwendigen Kraft von der ersten Handhabe 2 auf die Nadel 1 gewährleistet ist. Erfindungsgemäß weist das in Fig. 1 dargestellte Ausführungsbeispiel außerdem eine zweite Handhabe 3 auf, wobei diese Handhabe 3 auf der Nadel 1 in Längsrichtung der Nadel 1 verschiebbar angeordnet ist bzw. die Nadel 1 durch die zweite Handhabe 3 hindurch geschoben werden kann. Bei der konkreten Anwendung befindet sich die zweite Handhabe 3 in einer Position, in der sie sich regelmäßig mit ihrem dem Ende der Nadel 1 zugewandten Ende zumindest in der Nähe der Gewebeoberfläche des zu behandelnden Patienten befindet. Die Nadel 1 wird dabei unter Zuhilfenahme der ersten Handhabe 2 durch die an der Nadel 1 angreifenden, eine Steuerung des Eindringens der Nadel 1 ermöglichenden Handhabe 3 hindurch in das Gewebe vorgeschoben.

Bei dem dargestellten Ausführungsbeispiel weisen sowohl die erste Handhabe 2 als auch die zweite Handhabe 3 Verlängerungen 4, 5 auf, die an ein Angreifen an der Handhabe 2, 3 außerhalb des Strahlungsbereiches einer bildgebenden Diagnostik ermöglicht. Um einen besseren Griff an den Verlängerungen 4, 5 der Handhaben 2, 3 zu gewährleisten, sind bei dem dargestellten Ausführungsbeispiel in den Verlängerungen 4, 5 Öffnungen 6 vorgesehen.

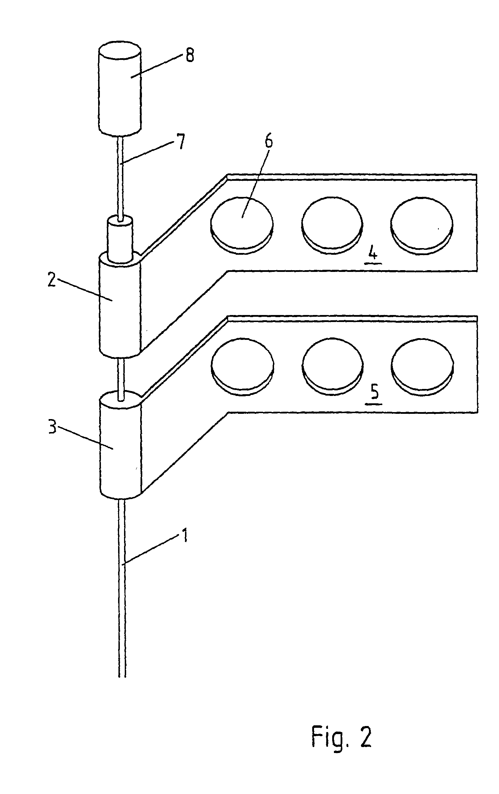

In Fig. 2 ist das in Fig. 1 dargestellte Ausführungsbeispiel in einer perspektivischen Ansicht mit der Nadel 1, der ersten Handhabe 2, der zweiten Handhabe 3 und den beiden Verlängerungen 4, 5 dargestellt. In Fig. 2 ist weiter dargestellt, daß in der Nadel 1 eine herausziehbare Innenkanüle 7 zum Verschluß der Nadel 1 beim Einführen in das Gewebe vorgesehen ist. Die Innenkanüle 7 ist an ihrem oberen Ende mit einer dritten Handhabe 8 versehen, die ein einfaches Herausziehen der Innenkanüle 7 aus der Nadel 1 gewährleistet.

## Patentansprüche

1. Interventionsvorrichtung mit einer Nadel (1) und einer ersten Handhabe (2), wobei die erste Handhabe (2) mit der Nadel (1) fest verbunden ist, sowie mit einer zweiten Handhabe (3),
**darduch gekennzeichnet, daß** die zweite Handhabe (3) beim Einführen in das Gewebe in Längsrichtung verschiebbar auf der Nadel (1) angeordnet ist.

2. Interventionsvorrichtung nach Anspruch 1,
**dadurch gekennzeichnet, daß** die erste und/oder zweite Handhabe (2, 3) radial zur Achse der Nadel (1) eine Verlängerung (4, 5) aufweisen.

3. Interventionsvorrichtung nach Anspruch 1 oder 2,
**dadurch gekennzeichnet, daß** das Material der ersten und/oder zweiten Handhabe (2, 3) transparent für elektromagnetische Strahlung ist.

4. Interventionsvorrichtung nach einem der Ansprüche 1 bis 3,
**dadurch gekennzeichnet, daß** die zweite Handhabe (3) einen Längsschlitz zum Einbringen der Nadel (1) in die axiale Führung der zweiten Handhabe (3) aus einer Richtung radial zur Achse der Nadel (1) aufweist.

5. Interventionsvorrichtung nach einem der Ansprüche 1 bis 4,
**dadurch gekennzeichnet, daß** in der Nadel eine entfernbare Innenkanüle (7) angeordnet ist.

6. Interventionsvorrichtung nach Anspruch 5,
**dadurch gekennzeichnet, daß** die Innenkanüle (7) an einer dritten Handhabe (8) befestigt ist und die dritte Handhabe (8) mit der ersten Handhabe (2) lösbar verbindbar ist.

## Claims

1. An intervention device comprising a needle (1) and a first handle (2), wherein the first handle (2) is fixedly connected to the needle (1), and comprising a second handle (3), **characterised in that** the second handle (3) is arranged displaceably in the longitudinal direction on the needle (1) during insertion into the tissue.

2. The intervention device according to claim 1, **characterised in that** the first and/or the second handle (2, 3) have an extension (4, 5) radial to the axis of the needle (1).

3. The intervention device according to claim 1 or claim 2, **characterised in that** the material of the first and/or the second handle (2, 3) is transparent to electromagnetic radiation.

4. The intervention device according to any one of claims 1 to 3, **characterised in that** the second handle (3) has a longitudinal slit for insertion of the needle (1) into the axial guide of the second handle (3) from a direction radial to the axis of the needle (1).

5. The intervention device according to any one of claims 1 to 4, **characterised in that** a removeable inner cannula (7) is arranged in the needle.

6. The intervention device according to claim 5, **characterised in that** the inner cannula (7) is affixed to a third handle (8) and the third handle (8) is detachably connectable to the first handle (2).

## Revendications

1. Dispositif d'intervention comprenant une aiguille (1) et une première manette (2), la première manette (2) étant reliée de manière fixe à l'aiguille (1), ainsi qu'une deuxième manette (3),
**caractérisé en ce que** la deuxième manette (3) est disposée de manière mobile dans le sens longitudinal sur l'aiguille (1) lors de l'introduction dans le tissu.

2. Dispositif d'intervention selon la revendication 1,
**caractérisé en ce que** la manière de la première et/ou de la deuxième manette (2, 3) présentent un prolongement (4, 5) radialement par rapport à l'axe de l'aiguille (1).

3. Dispositif d'intervention selon la revendication 1 ou 2,
**caractérisé en ce que** la première et/ou la deuxième manette (2, 3) est transparente au rayonnement électromagnétique.

4. Dispositif d'intervention selon l'une des revendications 1 à 3,
**caractérisé en ce que** la deuxième manette (3) présente une fente longitudinale pour mettre en place l'aiguille (1) dans le guidage axial de la deuxième manette (3) à partir d'une direction radialement par rapport à l'axe de l'aiguille (1).

5. Dispositif d'intervention selon l'une des revendications 1 à 4,
**caractérisé en ce qu'**une canule intérieure amovible (7) est disposée dans l'aiguille.

6. Dispositif d'intervention selon la revendication 5,
**caractérisé en ce que** la canule intérieure (7) est fixée sur une troisième manette (8) et la troisième manette (8) est reliée, de manière amovible, avec la première manette (2).
